# EUROPEAN PATENT SPECIFICATION

(11) **EP 2 873 664 B1**
(45) Date of publication and mention of the grant of the patent: **01.11.2017**
(21) Application number: 13817593.0
(22) Date of filing: 04.06.2013
(51) Int. Cl.: C07D 401/14, A61K 31/4709, A61P 35/00

(54) **CRYSTALLINE FORM I OF TYROSINE KINASE INHIBITOR DIMALEATE AND PREPARATION METHODS THEREOF**
KRISTALLINE FORM I DES TYROSINKINASEINHIBITORS DIMALEAT UND HERSTELLUNGSVERFAHREN DAFÜR
FORME CRISTALLISÉE I DU DIMALÉATE INHIBITEUR DE TYROSINE KINASE ET SES MÉTHODES DE PRÉPARATION

(30) Priority: 12.07.2012 CN 201210240697
(43) Date of publication of application: 20.05.2015
(73) Proprietor: Jiangsu Hengrui Medicine Co., Ltd., Jiangsu 222047 (CN)
(72) Inventor: SUN, Piaoyang, Lianyungang Jiangsu 222047 (CN); WU, Guaili, Lianyungang Jiangsu 222047 (CN); YUAN, Bo, Lianyungang Jiangsu 222047 (CN); CHEN, Yongjiang, Lianyungang Jiangsu 222047 (CN)
(74) Representative: Potter Clarkson LLP
(86) International application number: PCT/CN2013/076717
(87) International publication number: WO 2014/008794

(56) References cited:
- WO-A1-2011/029265
- WO-A2-2012/122865
- CN-A- 101 824 029
- CN-A- 101 918 390
- RICHARD J BASTIN ET AL: "Salt Selection and Optimisation Procedures for Pharmaceutical New Chemical Entities", ORGANIC PROCESS RESEARCH AND DEVELOPMENT, AMERICAN CHEMICAL SOCIETY, US, vol. 4, no. 5, 19 July 2000 (2000-07-19), pages 427-435, XP008154792, ISSN: 1083-6160, DOI: 10.1021/OP000018U [retrieved on 2000-07-19]

## Description

### FIELD OF THE INVENTION

The present invention relates to a crystalline form of dimaleate of tyrosine kinase inhibitors, particularly relates to form I crystal of (R, E)-N-(4-(3-chloro-4-(pyridin-2-yl-methoxy)phenylamino)-3-cyano-7-ethoxyquinolin-6 -yl)-3-(1-methylpyrrolidino-2-yl)acrylamide dimaleate and the preparation and use thereof.

### BACKGROUND OF THE INVENTION

In recent years, the cancer mortality in our country was clearly on the rise. People's life and quality of life were threatened seriously with cancer. Thus, it is a challenging and significant subject to search new anticancer drugs with high effect and low toxicity in the life sciences nowadays. Receptor tyrosine kinase is a kind of transmembrane protein involved in signal transduction. It is shown that over 50% of the proto-oncogene and oncogene product have the tyrosine kinase activity, whose abnormal expression will cause tumorigenesis. Tyrosine kinase inhibitor has been approved to be on the market since 2001, which has become a new class of anticancer drugs with outperformance.

Epidermal growth factor receptor (EGFR) is a member of the receptor tyrosine kinase family. The epidermal growth factor receptor pathway plays a very important role in the tumorigenesis and progression, which has become the main research subject and one of the developing targets in the field of cancer therapy. Such drugs which has been on the market now comprise erlotinib, gefitinib and lapatinib (Tykerb, GW572016), as well as neratinib which is in the clinical phase now. Patent WO2011029265 discloses the method for the preparation of the compound (R, E)-N-(4-(3-chloro-4-(pyridin-2-ylmethoxy)phenylamino)-3-cyano-7-ethoxyquino line-6-yl)-3-(1-methylpyrrolidino-2-yl)acrylamide (referred as SHR1258 below for convenience). This drug molecule has distinct advantage of pharmacokinetics and pharmacodynamics. Although patent WO2011029265 discloses the chemical structure and preparation method of compound SHR1258, but is silent to the condition of its salification. In CN102675287A , the dimaleate of this compound (referred as SHR1258 dimaleate below) has been described, and its structure is as follows:

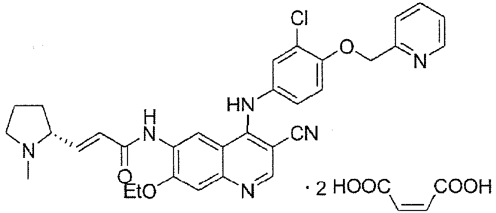

However, the inventors did not make further research for the polymorph and preparation method of SHR1258 dimaleate. As known for the person skilled in the art, the polymorph structure of the pharmaceutically active ingredient always affects the chemical stability of the drug. Different storage conditions and crystallization conditions may lead to the change in the polymorph structure of the compound, which is sometimes accompanied with other forms of polymorph. In general, amorphous drug product does not have regular crystal structure, which often has other defects such as poor product stability, smaller particle size, hard filtration, agglomerate easily, and poor liquidity. Thus, it is necessary to improve all aspects of the nature of the above product. It is need to search a new polymorph which has higher polymorph purity and better chemical stability.

### SUMMARY OF THE INVENTION

The present invention is to provide a stable crystal form of SHR1258 dimaleate and the preparation method thereof.

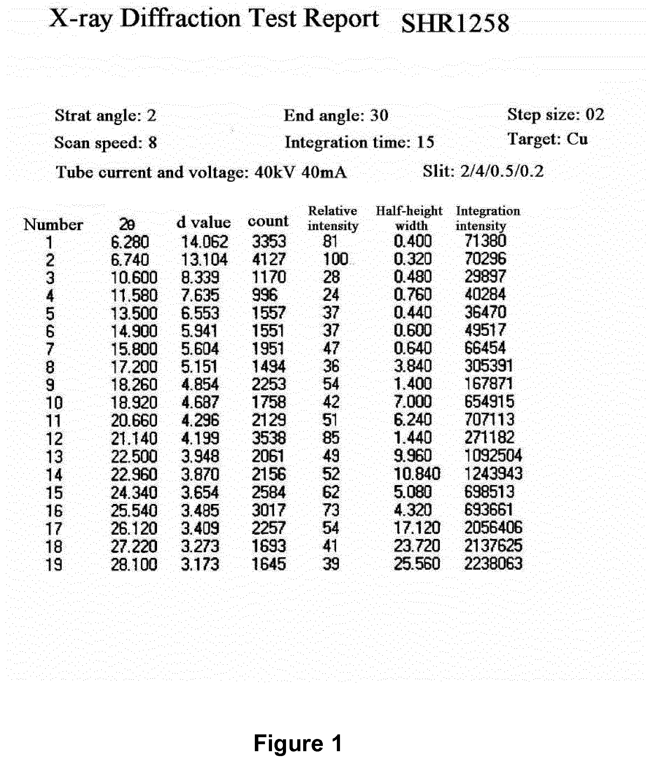

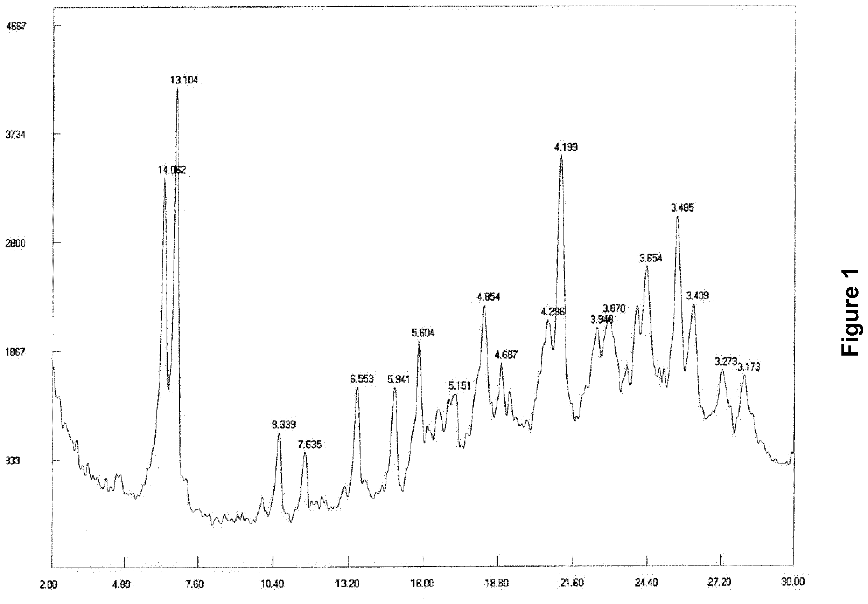

The inventor has test a series of crystallization products of SHR1258 dimaleate obtained under various conditions by X-ray diffraction and DSC test. The results prove that a stable crystal form of SHR1258 dimaleate which is referred as form I crystal can be obtained under normal crystallization condition. DSC patterns of the present form I crystal of SHR1258 dimaleate show distinct fusion absorption peak at 130°C. The X-ray diffraction pattern is shown in figure 1 which use Cu-Kα radiation to obtain the X-ray diffraction patterns represented by 2θ angels and interplannar crystal spacing (d value) in which there are characteristic peaks at 6.28(14.06), 6.74(13.10), 10.60(8.34), 11.58(7.64), 13.50(6.55), 14.90(5.94),15.80(5.60), 18.26(4.85), 20.66(4.30), 21.14(4.20), 22.96(3.87), 24.34(3.65), 25.54(3.49), 26.12(3.41).

In the method for the preparation of the crystal form I of the present invention, there is no special limitation for the existing form of SHR1258 dimaleate as starting material, and it can be used in any crystal form or amorphous form. The method for the preparation of form I crystal of SHR1258 dimaleate of the present invention comprises the following steps:
When use the low organic solvent, the polar organic solvent with fewer carbon atom and higher volatility which could be used as crystallization solvent is preferred, such as alcohols, ketones, esters, or the mixture thereof; isopropyl alcohol, acetone, ethanol, ethyl acetate, tetrahydrofuran or the mixture thereof is more preferred for the recrystallization of SHR1258 dimaleate. The solvent for crystallization can be a single solvent or a mixture solvent comprising the solvents mentioned above.

Specifically, the process for the preparation of form I crystal of SHR1258 dimaleate comprises the following steps:
(1)The mixture of SHR1258 and maleic acid or the SHR1258 dimaleate solid is dissolved in q.s. of organic solvents, and the solution obtained is cooled to crystallization.
(2) The crystal is filtered, washed and dried.

In a preferred embodiment of the present invention, the organic solvent in step 1 is selected from one or more solvent of alcohols with no more than three carbons, acetone, ethyl esters, tetrahydrofuran, preferably ethanol, isopropyl alcohol, tetrahydrofuran.

Furthermore, the most preferred single solvent is isopropyl alcohol.

In another embodiment of the present invention, the preferred mixture solvent is the mixture of ethanol and tetrahydrofuran. The ratio of the two is not limited, while the volume ratio of 1:1 is preferred in an embodiment of the present invention.

The recrystallization method is not limited and can be performed with conventional recrystallization process in the art, for example, it may be that the SHR1258 dimaleate is heated to dissolve in organic solvent, and then the solution is cooled gradually to stand to crystallize or the solution is stirred to crystallize; after crystallization the resulting precipitate is collected by filtration and then dried. In particular, a full conversion process is necessary for the formation of the stable crystal form, and amorphous structure or crystals with lower purity will form easily when the crystallization process is too fast which is usually caused by the supersaturation solution. The increase in solvent volume or the decrease in crystallization rate will be helpful for the formation of a stable crystal form with high purity. The crystal obtained by filtration is usually dried in vacuum at about 30∼100°C, preferred 40∼60°C, to remove recrystallization solvent.

The resulting crystal form of SHR1258 dimaleate was determined by DSC and X-ray diffraction patterns. Meanwhile, the residual solvent was also determined.

The crystal of SHR1258 dimaleate prepared according to the present method has no or very little residual solvent, which meets the requirement of the national pharmacopoeia for the residual solvent of drug products. Thus the crystal of the present invention can be properly used as pharmaceutical active ingredient.

The research results show that, the stability of the form I crystal of SHR1258 dimaleate obtained in the present invention is significantly better than the amorphous form under the condition of high temperature and high humidity. Moreover, the form I crystal has good stability under the condition of grinding, pressure and heating which can meet the production, transportation and storage requirements of medicaments. The preparation process is stable and repeatable, which is especially suitable for the industrial production.

### BRIEF DESCRIPTION OF THE DRAWINGS

Figure 1 shows the X-ray powder diffraction pattern for the form I crystal of SHR1258 dimaleate.
Figure 2 shows the Differential Scanning Calorimetry pattern for the form I crystal of SHR1258 dimaleate.
Figure 3 shows the X-ray powder diffraction pattern for amorphous form of SHR1258 dimaleate.
Figure 4 shows the Differential Scanning Calorimetry pattern for amorphous form of SHR1258 dimaleate.

### DETAILED DESCRIPTION OF THE INVENTION

The present invention is illustrated by the following examples in detail which in no way should be construed as limiting the scope of the present invention.

### Experimental instruments

### 1. Thermal analysis (DSC)

Instrument type: Perkin-Elmer Pyris 1 Series Thermal Analysis System
Purging gas: Nitrogen
Heating rate: 10.0°C/min
Temperature range: 50-300 °C

### 2. X-ray diffraction spectrum

Instrument type: D/Max-RA Japan rigaku X-ray powder diffraction
Ray: monochromatic Cu- K rays ( =1.5418 Å)
Scanning mode: /2 , Angular scan of 2-40°
Voltage: 40KV, Electric Current:40mA

### Example 1

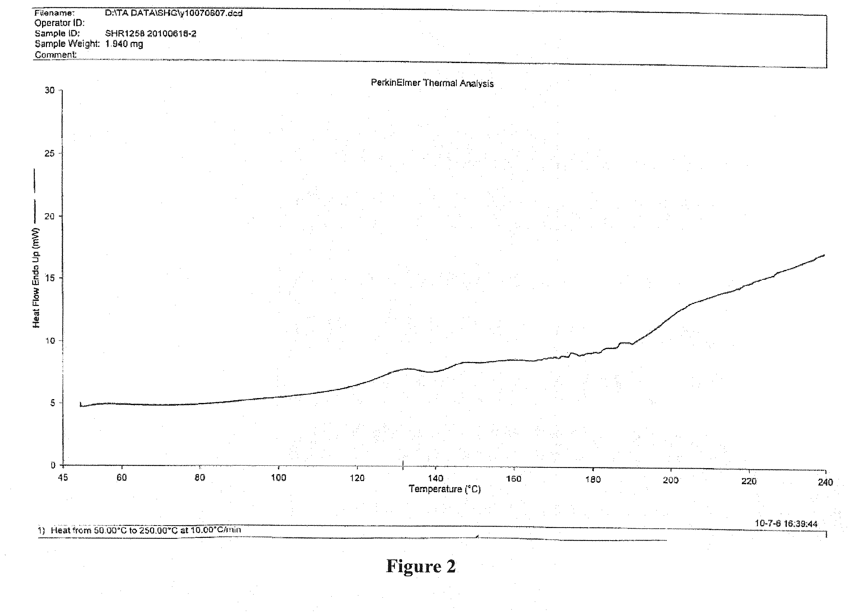

1.0g of SHR1258 (prepared according to patent WO2011029265) and 0.4g of maleic acid were dissolved in 25ml of isopropyl alcohol by heating. A solid was present while refluxing. After removing from heating, the obtained mixture was stirred to precipitate. The resulting precipitate was collected by filtration and then dried at 45 °C in vacuum overnight to obtain 0.85g of SHR1258 dimaleate crystal. Yield: 60%. X-ray diffraction pattern is shown in figure 1 in which there are characteristic peaks at 6.28 (14.06), 6.74(13.10),10.60(8.34), 11.58(7.64), 13.50(6.55), 14.90(5.94), 15.80(5.60), 18.26 (4.85), 20.66 (4.30), 21.14 (4.20), 22.96(3.87), 24.34 (3.65), 25.54 (3.49), 26.12 (3.41). DSC pattern is shown in figure 2, with sharp molten absorption peak at 131.429°C. The crystal was defined as form I crystal.

### Example 2

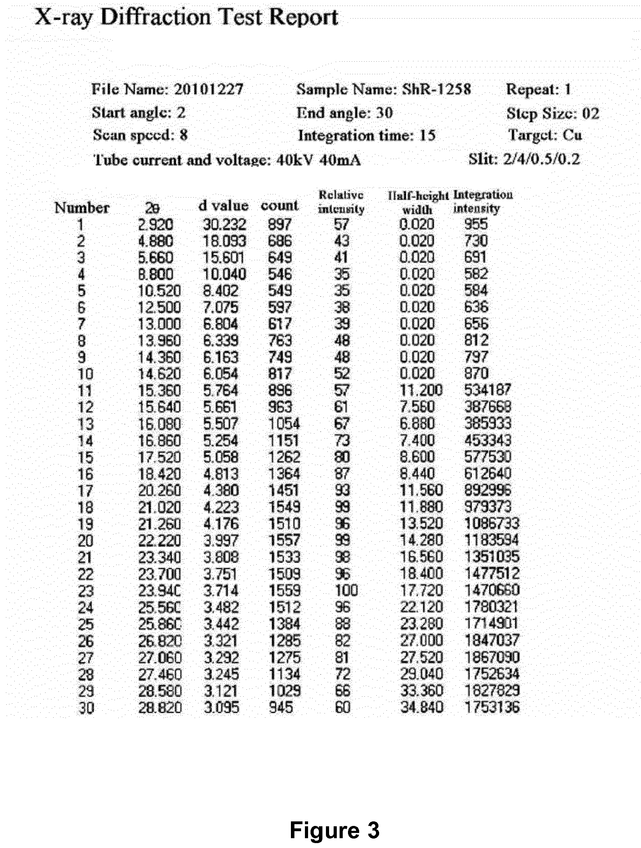

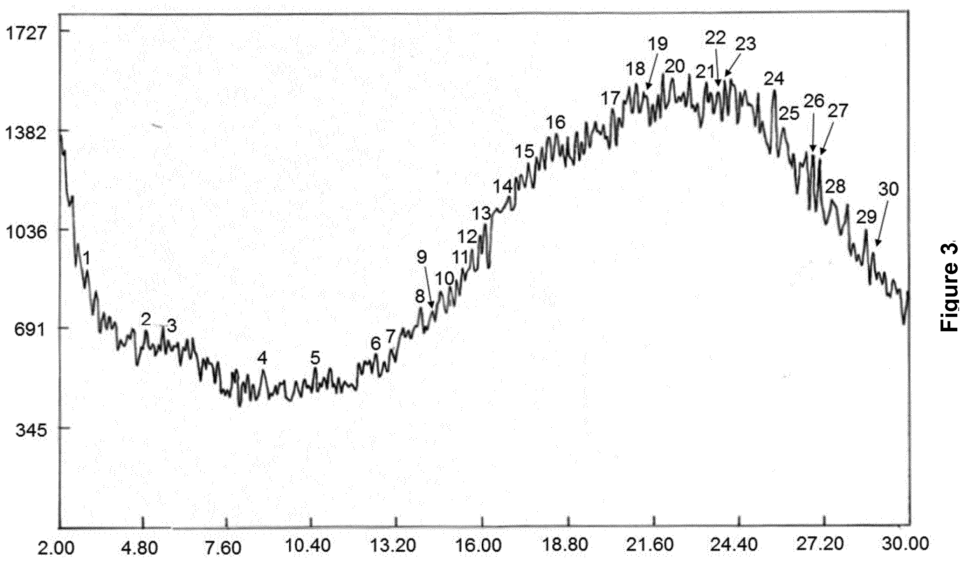

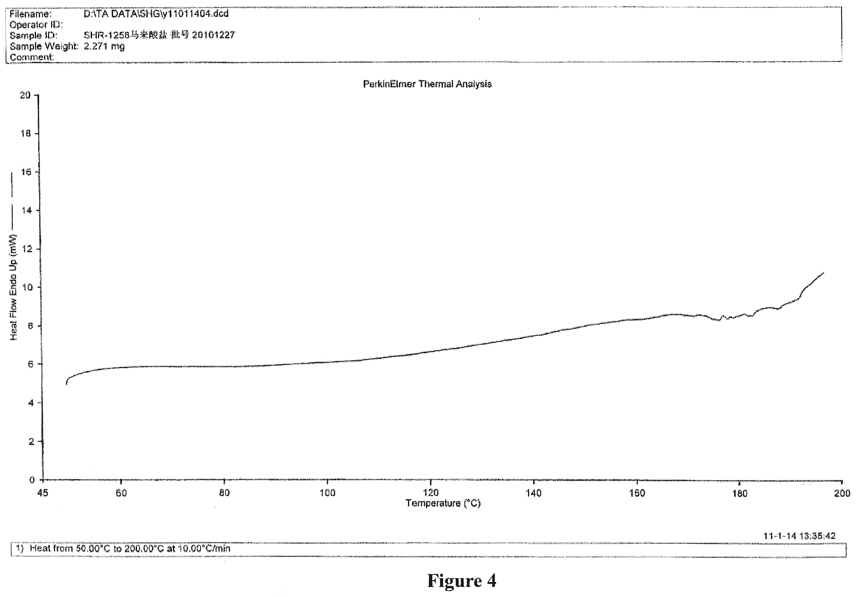

1.0g of SHR1258 and 0.4g of maleic acid were dissolved in 20ml of ethanol by heating. After removing from heating, the mixture was stirred overnight (the solid seperated was sticky). The next day, the mixture was added with 30ml of diethyl ether and stirred. The resulting precipitate was collected by filtration, washed with diethyl ether and then dried to obtain 1.03g of yellow solid. Yield: 73.5%. X-ray diffraction pattern of this solid is shown in figure 3 in which there are no characteristic peaks. DSC pattern is shown in figure 4, with no molten absorption peak below 170°C. It was determined that the product was amorphous form.

### Example 3

1.0g of SHR1258 dimaleate (prepared according to example 2) was added with 5ml of methanol and the mixture was heated to reflux until the solution obtained. The solvent was removed by evaporation in vacuum and 20ml of isopropyl alcohol was added. The solid was dissolved completely by heating and some solid presented while refluxing. After removing from heating, the mixture was laid to crystallization. The precipitate was collected by filtration and dried to obtain 0.80g solid. Yield: 80.0%. It was determined as form I crystal of SHR1258 dimaleate after comparing the X-ray diffraction patterns and DSC patterns.

### Example 4

2.0g of SHR1258 and 0.8g of maleic acid were heated to dissolve in 26ml of ethanol and tetrahydrofuran mixture (at a volume ratio of 1:1). The solution was stirred in 45°C water bath with solid seperated. After removing from heating, the mixture was stirred to crystallization. The precipitate was collected by filtration and dried at 45°C in vacuum overnight to obtain 2.3g of crystal. Yield: 82.0%. It was determined as form I crystal of SHR1258 dimaleate after comparing the X-ray diffraction patterns and DSC patterns.

### Example 5

1.0g of SHR1258 dimaleate solid (prepared according to example 2) was added in 5ml of water. The mixture was heated to reflux until the solution obtained. The solution was stirred to precipitate and sticky solid appeared the next day. The precipitate was collected by filtration and dried to obtain 0.68g solid. Yield: 68.3%. It was determined as the amorphous form of SHR1258 dimaleate from the X-ray diffraction patterns and DSC patterns.

### Example 6

The form I crystal of SHR1258 dimaleate prepared in example 1 and the amorphous form of SHR1258 dimaleate prepared in example 2 were placed opening in the air to test the stability in various conditions including illumination (4500Lux), heating (60°C), humility (RH90%). The investigation duration is five and ten days and the HPLC analysis results are shown in table 1.

**Table 1. The comparing of stability of form I crystal and amorphous form crystal of SHR1258 dimaleate**

| Bach number | Solvent | Time (Day) | Light | 60 °C | RH90% |
|---|---|---|---|---|---|
| Form I Crystal | Isopropyl Alcohol | 0 | 99.65% | 99.65% | 99.65% |
| | | 5 | 98.20% | 98.13% | 98.35% |
| | | 10 | 97.91% | 96.86% | 98.35% |
| Amorphous Form | 95% Ethyl Alcohol | 0 | 98.41% | 98.41% | 98.41% |
| | | 5 | 96.96% | 95.24% | 96.95% |
| | | 10 | 96.91% | 93.71% | 96.31% |

The form I crystal of SHR1258 dimaleate and the amorphous form of SHR1258 dimaleate were placed opening in the air in various conditions including illumination, heating and humility. The results show that the stability of the form 1 crystal of SHR1258 dimaleate and amorphous form of SHR1258 dimaleate are similar under the light without statistically significant. The form I crystal of SHR1258 dimaleate is more stable than amorphous SHR1258 dimaleate under high temperate and high moisture condition.

### Example 7

The form I crystal of SHR1258 dimaleate prepared in example 1 was grinded, heated and pressed, then judged by X-ray diffraction and DSC patterns. The results show that the crystal is stable and the data is shown in table 2.

**Table 2**

| Batch number | Process | Experience process | Crystal | DSC |
|---|---|---|---|---|
| Experiment 7.1 S0915100402G | Grinding for 10 min | 1.0g of form I crystal of SHR1258 dimaleate was grinded for 10min in mortar under nitrogen atmosphere. | Form I | DSC peak: 130.716 °C |
| Experiment 7.2 S0915100402H | Heating at 80°C for 3 hours | 1.0g of form I crystal of SHR1258 dimaleate was spread and heated at 80°C for 3 hours | Form I | DSC peak: 133.588 °C |
| Experiment 7.3 S0915100402P | Pressing | Pressing the form I crystal of SHR1258 dimaleate into pieces | Form I | DSC peak: 131.726 °C |

## Claims

1. Form I crystal of (R,E)-N-(4-(3-chloro-4-(pyridin-2-ylmethoxy)-phenylamino)-3-cyano-7-ethoxyquinolin-6-yl)-3-(1-methylpyrrolidin-2-yl) acrylamide dimaleate **characterized in that** using Cu-Kα radiation to obtain the X-ray diffraction patterns represented by 2θ angles and interplanar crystal spacing, the crystal has X-ray diffraction patterns as shown in figure 1 in which there are characteristic peaks at 6.28(14.06), 6.74(13.10), 10.60(8.34),11.58 (7.64),13.50(6.55), 14.90(5.94), 15.80(5.60), 18.26(4.85), 20.66(4.30),21.14(4.20),22.96(3.87),24.34(3.65),25.54(3.49),and 26.12(3.41).

2. A method for the preparation of form I crystal of (R,E)-N-(4-(3-chloro-4-(pyridin-2-ylmethoxy)-phenylamino)-3-cyano-7- ethoxyquinolin-6-yl)-3-(1-methylpyrrolidin-2-yl)acrylamide dimaleate as claimed in claim 1 which comprises the following steps:
1) The mixture of any crystal form or amorphous form of (R,E)-N-(4-(3- chloro-4-(pyridin-2-ylmethoxy)-phenylamino)-3-cyano-7-ethoxyquinolin-6-yl)-3-(1-methylpyrrolidin-2-yl)acrylamide and maleic acid, or the solid of any crystal form or amorphous form of (R,E)-N-(4-(3-chloro-4- (pyridin-2-ylmethoxy)-phenylamino)-3-cyano-7-ethoxyquinolin-6-yl)-3-(1-methylpyrrolidin-2-yl)acrylamide dimaleate, is heated to dissolve in quantum sufficit of organic solvent, then cooled to crystallization; said organic solvent is one or more of the solvent selected from the alcohol with no more than three carbons, acetone, ethyl acetate, tetrahydrofuran, preferably ethanol, isopropyl alcohol, tetrahydrofuran;
2) The crystal was filtered, washed and dried.

3. The method as claimed in claim 2, **characterized in that** the organic solvent in step 1) is isopropyl alcohol.

4. The method as claimed in claim 2, **characterized in that** the organic solvent in step 1) is mixture solvent of ethanol and tetrahydrofuran.

5. A pharmaceutical composition comprising form I crystal of (R,E)-N-(4-(3-chloro-4-(pyridin-2-ylmethoxy)-phenylamino)-3-cyano-7- ethoxyquinolin-6-yl)-3-(1-methylpyrrolidin-2-yl)acrylamide dimaleate according to claim 1 and pharmaceutically acceptable carrier.

6. Use of form I crystal as claimed in claim 1 or pharmaceutical composition as claimed in claim 5 in the preparation of a medicament for the treatment of disease relevant to protein kinase, wherein said the protein kinase is chosen from EGFR protein tyrosine kinases or HER-2 receptor protein tyrosine kinases.

7. Use of form I crystal as claimed in claim 1 or pharmaceutical composition as claimed in claim 5 in the preparation of a medicament for the treatment of cancer, wherein said cancer is lung cancer, breast cancer, epidermal squamous carcinoma or gastric cancer.

8. Form I crystal as claimed in claim 1 or pharmaceutical composition as claimed in claim 5 for use in the treatment of disease relevant to protein kinase, wherein said the protein kinase is chosen from EGFR protein tyrosine kinases or HER-2 receptor protein tyrosine kinases.

9. Form I crystal as claimed in claim 1 or pharmaceutical composition as claimed in claim 5 for use in the treatment of cancer, wherein said cancer is lung cancer, breast cancer, epidermal squamous carcinoma or gastric cancer.

## Patentansprüche

1. Kristall der Form I aus (R,E)-N-(4-(3-Chlor-4-(pyridin-2-ylmethoxy)-phenylamino)-3-cyano-7-ethoxychinolin-6-yl)-3-(1-methylpyrrolidin-2-yl)-acrylamiddimaleat, **dadurch gekennzeichnet, dass** Cu-Kα-Strahlung eingesetzt wird, um die Röntgendiffraktionsmuster zu erhalten, die dargestellt werden durch 20 Grad und interplanaren Kristallabstand, wobei der Kristall Röntgendiffraktionsmuster wie in Figur 1 dargestellt aufweist, wo charakteristische Spitzen bei 6,28 (14,06), 6,74 (13,10), 10,60 (8,34),11,58 (7,64),13,50 (6,55), 14,90 (5,94), 15,80 (5,60), 18,26 (4,85), 20,66 (4,30), 21,14 (4,20), 22,96 (3,87), 24,34 (3,65), 25,54 (3,49) und 26,12 (3,41) vorliegen.

2. Verfahren zum Erzeugen eines Kristalls der Form I aus (R,E)-N-(4-(3-Chlor-4-(pyridin-2-ylmethoxy)-phenylamino)-3-cyano-7-ethoxychinolin-6-yl)-3-(1-methylpyrrolidin-2-yl)acrylamiddimaleat nach Anspruch 1, das die folgenden Schritte umfasst:
1) Das Gemisch einer beliebigen Kristallform oder amorphen Form von (R,E)-N-(4-(3-Chlor-4-(pyridin-2-ylmethoxy)-phenylamino)-3-cyano-7-ethoxychinolin-6-yl)-3-(1-methylpyrrolidin-2-yl)acrylamid und Maleinsäure oder der Feststoff einer beliebigen Kristallform oder amorphen Form von (R,E)-N-(4-(3-Chlor-4-(pyridin-2-ylmethoxy)-phenylamino)-3-cyano-7-ethoxychinolin-6-yl)-3-(1-methylpyrrolidin-2-yl)acrylamiddimaleat wird erwärmt, um in einer ausreichenden Menge in einem organischen Lösungsmittel gelöst zu werden und dann abgekühlt zum Kristallisieren; das organische Lösungsmittel ist ein oder mehrere Lösungsmittel, ausgewählt aus dem Alkohol mit nicht mehr als drei Kohlenstoffen, Aceton, Ethylacetat, Tetrahydrofuran, vorzugsweise Ethanol, Isopropylalkohol, Tetrahydrofuran;
2) Der Kristall wurde gefiltert, gewaschen und getrocknet.

3. Verfahren nach Anspruch 2, **dadurch gekennzeichnet, dass** das organische Lösungsmittel in Schritt 1) Isopropylalkohol ist.

4. Verfahren nach Anspruch 2, **dadurch gekennzeichnet, dass** das organische Lösungsmittel aus Schritt 1) ein Mischlösungsmittel aus Ethanol und Tetrahydrofuran ist.

5. Pharmazeutische Zusammensetzung, umfassend einen Kristall der Form I aus (R,E)-N-(4-(3-Chlor-4-(pyridin-2-ylmethoxy)-phenylamino)-3-cyano-7-ethoxychinolin-6-yl)-3-(1-methylpyrrolidin-2-yl)acrylamiddimaleat nach Anspruch 1 und einen pharmazeutisch unbedenklichen Träger.

6. Gebrauch des Kristalls der Form I nach Anspruch 1 oder der pharmazeutischen Zusammensetzung nach Anspruch 5 beim Erzeugen eines Medikaments zum Behandeln einer Erkrankung in Verbindung mit Proteinkinase, wobei die Proteinkinase ausgewählt ist aus EGFR-Proteintyrosinkinasen oder HER-2-Rezeptor-Proteintyrosinkinasen.

7. Gebrauch eines Kristalls der Form I nach Anspruch 1 oder einer pharmazeutischen Zusammensetzung nach Anspruch 5 beim Erzeugen eines Medikaments zum Behandeln von Krebs, wobei der Krebs Lungenkrebs, Brustkrebs, Plattenepithelkarzinom der Epidermis oder Magenkrebs ist.

8. Kristall der Form I nach Anspruch 1 oder pharmazeutische Zusammensetzung nach Anspruch 5 für den Gebrauch beim Behandeln einer Erkrankung in Verbindung mit Proteinkinase, wobei die Proteinkinase ausgewählt ist aus EGFR-Proteintyrosinkinasen oder HER-2-Rezeptor-Proteintyrosinkinasen.

9. Kristall der Form I nach Anspruch 1 oder pharmazeutische Zusammensetzung nach Anspruch 5 für den Gebrauch beim Behandeln von Krebs, wobei der Krebs Lungenkrebs, Brustkrebs, Plattenepithelkarzinom der Epidermis oder Magenkrebs ist.

## Revendications

1. Forme cristalline I du dimaléate de (R,E)-N-(4-(3-chloro-4-(pyridin-2-ylméthoxy)-phénylamino)-3-cyano-7-éthoxyquinoléin-6-yl)-3-(1-méthylpyrrolidin-2-yl)acrylamide, **caractérisée en ce qu'**à l'utilisation de la radiation de Cu-Kα pour obtenir les profils de diffraction des rayons X représentés par des valeurs d'angle 2θ et distances inter-réticulaires cristallines, elle produit le profil de diffraction des rayons X illustré à la Figure 1 comprenant les raies caractéristiques suivantes : 6,28 (14,06), 6,74 (13,10), 10,60 (8,34), 11,58 (7,64), 13,50 (6,55), 14,90 (5,94), 15,80 (5,60), 18,26 (4,85), 20,66 (4,30), 21,14 (4,20), 22,96 (3,87), 24,34 (3,65), 25,54 (3,49) et 26,12 (3,41).

2. Méthode de préparation de la forme cristalline I du dimaléate de (R,E)-N-(4-(3-chloro-4-(pyridin-2-ylméthoxy)-phénylamino)-3-cyano-7-éthoxyquinoléin-6-yl)-3-(1-méthylpyrrolidin-2-yl)acrylamide selon la revendication 1, qui comprend les étapes suivantes :
1) Le mélange de toute forme cristalline ou forme amorphe du (R,E)-N-(4-(3-chloro-4-(pyridin-2-ylméthoxy)-phénylamino)-3-cyano-7-éthoxyquinoléin-6-yl)-3-(1-méthylpyrrolidin-2-yl)acrylamide et d'acide maléique ou le solide de toute forme cristalline ou forme amorphe du dimaléate de (R,E)-N-(4-(3-chloro-4-(pyridin-2-ylméthoxy)-phénylamino)-3-cyano-7-éthoxyquinoléin-6-yl)-3-(1-méthylpyrrolidin-2-yl)acrylamide est chauffé dans une quantité suffisante d'un solvant organique pour être dissous avant d'être refroidi jusqu'à cristallisation ; ledit solvant organique est un ou plusieurs solvants choisis parmi un alcool ayant au plus trois carbones, l'acétone, l'acétate d'éthyle et le tétrahydrofurane, de préférence l'éthanol, l'alcool isopropylique et le tétrahydrofurane ;
2) Le cristal a été filtré, lavé et séché.

3. Méthode selon la revendication 2, **caractérisée en ce que** le solvant organique utilisé à l'étape 1) est l'alcool isopropylique.

4. Méthode selon la revendication 2, **caractérisée en ce que** le solvant organique utilisé à l'étape 1) est un mélange de solvants composé d'éthanol et de tétrahydrofurane.

5. Composition pharmaceutique comprenant la forme cristalline I du dimaléate de (R,E)-N-(4-(3-chloro-4-(pyridin-2-ylméthoxy)-phénylamino)-3-cyano-7-éthoxyquinoléin-6-yl)-3-(1-méthylpyrrolidin-2-yl)acrylamide selon la revendication 1 et un véhicule pharmaceutiquement acceptable.

6. Utilisation de la forme cristalline I selon la revendication 1 ou d'une composition pharmaceutique selon la revendication 5 dans la préparation d'un médicament pour le traitement d'une maladie qui fait intervenir une protéine kinase, où ladite protéine kinase est choisie parmi des protéines tyrosine kinases de l'EGFR ou des protéines tyrosine kinases du récepteur HER-2.

7. Utilisation de la forme cristalline I selon la revendication 1 ou d'une composition pharmaceutique selon la revendication 5 dans la préparation d'un médicament pour le traitement d'un cancer, où ledit cancer est un cancer du poumon, un cancer du sein, un carcinome épidermoïde à cellules squameuses ou un cancer de l'estomac.

8. Forme cristalline I selon la revendication 1 ou composition pharmaceutique selon la revendication 5 pour une utilisation dans le traitement d'une maladie qui fait intervenir une protéine kinase, où ladite protéine kinase est choisie parmi des protéines tyrosine kinases de l'EGFR ou des protéines tyrosine kinases du récepteur HER-2.

9. Forme cristalline I selon la revendication 1 ou composition pharmaceutique selon la revendication 5 pour une utilisation dans le traitement d'un cancer, où ledit cancer est un cancer du poumon, un cancer du sein, un carcinome épidermoïde à cellules squameuses ou un cancer de l'estomac.
